Europäisches Patentamt

(19) European Patent Office

Office européen des brevets

(11) Publication number: **0 346 739 B1**

## EUROPEAN PATENT SPECIFICATION

(12)

(45) Date of publication of patent specification: **08.09.93**  (51) Int. Cl.5: **B01D  61/36**, B01D 53/22, C07C 29/76

(21) Application number: **89110237.8**

(22) Date of filing: **06.06.89**

(54) Method for separating a liquid component from a solution containing two or more liquid components.

(30) Priority: **15.06.88 JP 148895/88**

(43) Date of publication of application:
**20.12.89 Bulletin  89/51**

(45) Publication of the grant of the patent:
**08.09.93 Bulletin  93/36**

(84) Designated Contracting States:
**DE FR GB NL**

(56) References cited:
**EP-A- 0 226 216**
**EP-A- 0 273 267**
**DE-C- 851 048**
**FR-A- 1 001 922**
**FR-A- 1 529 382**

**JOURNAL OF MEMBRANE SCIENCE, vol. 16, nos. 1-3, December 1983, pages 269-284, Amsterdam, NL; M.H.V. MULDER: "Ethanol-Water Separation by Pervaporation"**

(73) Proprietor: **Lignyte Co., Ltd.**
**1-4-62, Chibune Nishiyodogawa-ku**
**Osaka(JP)**

(72) Inventor: **Uragami, Tadashi**
**6-27-9, Aomadani-Higashi**
**Mino-shi Osaka(JP)**

(74) Representative: **Goddar, Heinz J., Dr. et al**
**FORRESTER & BOEHMERT Franz-Joseph-Strasse 38**
**D-80801 München (DE)**

EP 0 346 739 B1

**Description**

TECHNICAL FIELD

The present invention relates to a method for separating a liquid component from a solution containing two or more liquid components through the use of a permeable membrane, and more particularly to an improved method of separating the component in vapor-to-vapor permeation environment.

BACKGROUND ART

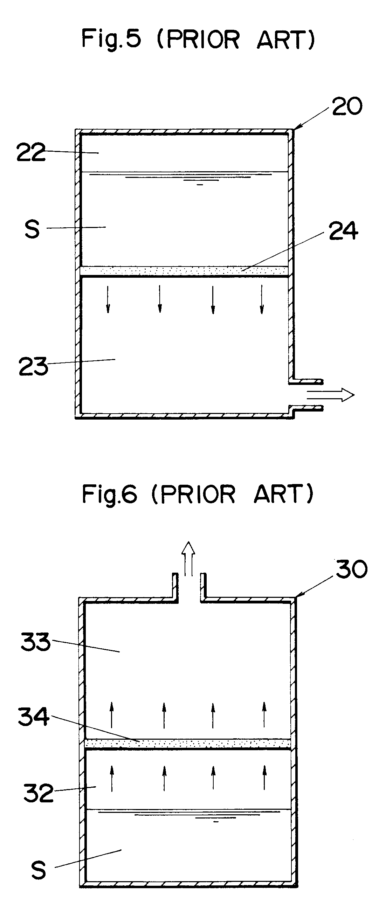

As a method of separating a particular liquid component from a solution or mixture such as an organic liquid containing two or more liquid components there has been developed a pervaporation (permeation evaporation) method. The principle of the permeation method will be briefly discussed with reference to FIG. 5 which employs a separation vessel **20** divided into an upper solution chamber **22** and a lower vacuum chamber **23** by means of a permeable membrane **24**. By reducing a pressure in the vacuum chamber **23** while keeping the solution **S** introduced into the solution chamber **22** in contact with the membrane **24**, a particular component in the liquid solution **S** will permeate and diffuse through the permeable membrane **24** selectively in preference to the other components so that the component to be separated selectively permeates through the membrane **24** and evaporate from the surface of the permeable membrane **24** into the vacuum chamber **23**. However, in this permeation method in which the liquid solution **S** is kept in direct contact with the permeable membrane **24** to effect the permeation in a liquid-to-vapor phase environment, there arises a serious problem that the permeable membrane **34** which is generally made from a macromolecular material is likely to be swelled due to the direct contact with the solution **S**. When the permeable membrane is swelled, its permeability will be considerably degraded to thereby lower the separation efficiency.

To overcome the above problem, the inventor has proposed in the prior, not pre-published European application No. 0 273 267 A2 an evapomeation (evaporation permeation) method which is directed to effect the permeation in vapor-to-vapor phase environment. That is, as schematically shown in FIG. 6, the evapomeation method utilizes a like vessel **30** divided into a lower solution chamber **32** and an upper vacuum chamber **33** by a permeable membrane **34** and effects the permeation separation while keeping the solution **S** from which a particular component is to be separated out of contact with the permeable membrane **34**. Thus, the vapors of the particular component will selectively permeate through the membrane **34** in a vapor phase into the vacuum chamber **33** when drawn by vacuum through the membrane **34**. With this result, the evapomeation method can be free from the swelling which is seen in the conventional pervaporation method and therefore prevents a lowering in separation efficiency. Nevertheless, the evapomeation method may be found insufficient in some cases due to limited separation efficiency inherent to the permeable membrane employed.

A process for the dehydration of a water containing medium is known from DE-C-0 851 048, wherein any contact between the water and the membrane is to be avoided and difference between the partial pressures of the water vapor on the inner and on the outer side of the container is caused, the latter being the lower. However, the membrane is always maintained on a higher temperature as the dew point of the water vapor on the inner side of the membrane.

In Journal of Membrane Science, Vol. 16, Nrs. 1-3, pages 269-284, the ethanol-water separation by pervaporation is described in its basic principles without any hint to the solution of the above mentioned problems. A further specific pervaporation process with specific membranes for specific purposes is described in FR-A-1529382.

DISCLOSURE OF THE INVENTION

The present invention eliminates the above problems and provides an improved permeation separation method for further enhancing separation efficiency. The method of the present invention utilizes a vessel which is divided by a permeable membrane into a solution chamber and a vacuum chamber. The permeable membrane is a membrane selectively and preferentially permeable to the vapors of the component which is intended to be separated and which has a lower boiling point than those of the other components. The solution is placed in the solution chamber out of direct contact with the permeable membrane. Then, a vacuum is applied to the vacuum chamber to evaporate the solution in the solution chamber and draw the resulting vapors through the permeable membrane into the vacuum chamber, whereby the components to be separated selectively separates through the membrane in a vapor-to-vapor

EP 0 346 739 B1

phase environment. The improvement resides in heating the solution in the solution chamber to a temperature higher than the environment while said zone adjacent to the solution chamber side of said membrane or said membrane itself is cooled to a temperature lower than that of said solution in the solution chamber. According to the improved method of providing a temperature difference between the solution and the permeable membrane or the zone adjacent thereto, a remarkable increase in separation efficiency will result. Although an exact mechanism or reason for the increased separation efficiency is not yet known, it is assumed that when reaching a cooled zone adjacent to the permeable membrane the vapors of the components not intended or having a higher boiling point are likely to aggregate more than those of the intended component or having a lower boiling point to thereby form a greater aggregation of molecules which is difficult to enter the membrane or even return back to the liquid phase. Thus, the vapors of the intended component having the lower boiling point are allowed to preferentially enter or permeate through the membrane while those of the other components are rejected so that the vapors of the intended component can be collected at a greater amount in the vacuum chamber than those of the components not intended.

Accordingly, it is a primary object of the present invention to provide an improved permeation separation method in which a particular liquid component can be separated from solution containing two or more liquid components at an increased separation efficiency.

The above and other objects and advantages of the present invention will become more apparent from the following description of the invention with reference to the attached drawings.

BRIEF DESCRIPTION OF THE DRAWINGS

FIG. 1 is a schematic view showing an apparatus employed for the present invention;

FIGS. 2 to 4 are respectively graphs illustrating separation efficiency and concentration of ethanol separated with differing temperature conditions from a 10 % aqueous solution of ethanol with respect to 9 (nine) samples in accordance with Example 1 of the present invention;

FIG. 5 is a schematic view illustrating a prior pervaporation method; and

FIG. 6 is a schematic view illustrating a basic concept of an evapomeation method.

MODES FOR CARRYING OUT THE INVENTION

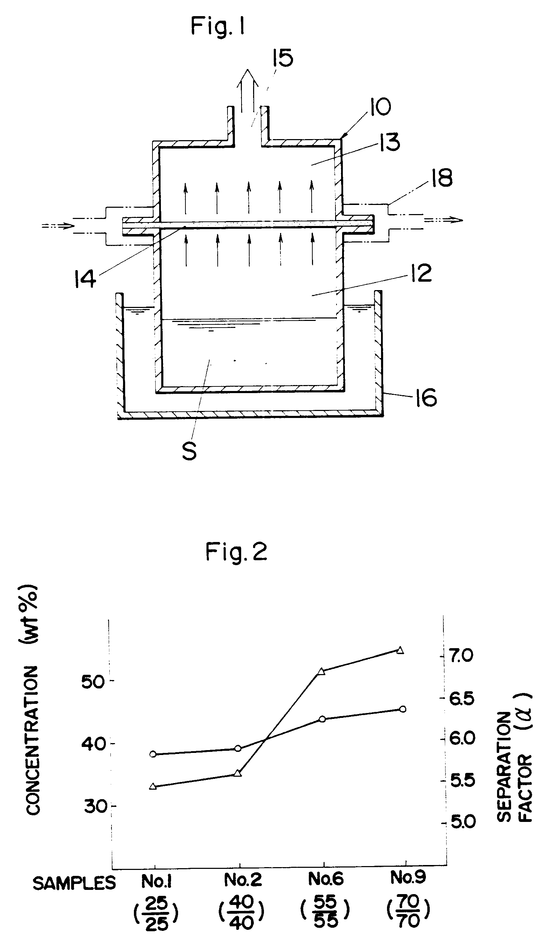

Referring to FIG. 1 which illustrates the principle of the "evapomeation (evaporation permeation)" separation method of the present invention, a separation vessel **10** is divided by means of a permeable membrane **14** into a lower solution chamber **12** and an upper vacuum chamber **13**. A solution **S**, for example, an organic solution containing two or more volatile or evaporizable components having different boiling points is introduced into the solution chamber **12** and is kept out of direct contact with the permeable membrane **14**. The vacuum chamber **13** is provided with a port **15** which is connected to a source of vacuum such as a vacuum pump for evacuating the vacuum chamber and in turn for evaporating the solution **S** in the solution chamber **12** and drawing the resulting vapors through the permeable membrane **14**. The vapors permeating through the membrane **14** into the vacuum chamber **13** is collected or recovered by a suitable manner. Since the permeable membrane **14** allows a particular component to selectively permeate in preference to the others, it is readily possible to complete the separation either by collecting the component having passed into the vacuum chamber **13** through the permeable membrane **14** or by collecting the remaining solution in the solution chamber **12** from which the particular component has been removed.

The permeable membrane **14** utilized in the present invention may be a membrane which is generally called a non-porous membrane including those utilized in the conventional pervaporation method. For example, the permeable membrane may be made from alginic acid, chitosan, cross-linked chitosan, chitosan acetate, quaternized chitosan, polystyrene, polyvinylidenechloride, polydimethylsiloxane, polyvinylidenefluoride, cellulose nitrate, cellulose acetate, cross-linked polyvinylalcohol, polyamic acid, poly-1-trimethylsilyl-1-propyne, graft copolymer of stylenedimethylsiloxane.

In the present invention, the permeable membrane is selected to be permeable preferentially to a liquid component having a lowest boiling point among the others in a solution. For instance, when an aqueous solution of alcohol, e.g. ethanol (b.p. 78.3 °C) or methanol (b.p. 64.1 °C) is intended to be separated, a permeable membrane of polydimethylsiloxane, poly-1-trimethylsilyl-1-propyne, or graft copolymer of stylene-dimethylsiloxane is selected to be permeable to alcohol in preference to water. For an aqueous solution of dimethylsulfoxide (b.p. 189 °C), dimethylformamide (b.p. 153 °C), dimethylacetamide (b.p. 165.5 °C), or acetic acid (b.p. 117.8 °C), a permeable membrane of alginic acid, chitosan, cross-linked

3

EP 0 346 739 B1

chitosan, cross-linked polyvinylalcohol, or polyamic acid is selected to be permeable to water in preference to the other component.

The permeable membrane **14** is normally provided as a thin fragile membrane so that it is difficult to withstand a pressure difference developed thereacross at the time of evacuating the vacuum chamber **13**. For reinforcement and prevention of the break of the permeable membrane, it is preferred to support the membrane by means of a porous member such as non-woven fabric of polypropylene or polyester, porous film of Teflon or polysulfone, porous glass plate, or porous ceramic plate.

In operation, as a vacuum is applied to the vacuum chamber **13**, the solution chamber **13** is correspondingly evacuated to vaporize the liquid components in the solution **S**. The resulting vapors are drawn to the vacuum chamber **12** through the permeable membrane **14**. When reaching the membrane **14**, the vapors of the component having a lower boiling point than the other are caused to permeate and diffuse preferentially through the membrane **14** and are eventually drawn into the vacuum chamber **13**. For example, when ethanol is intended to be selected from its aqueous solution by a suitable permeable membrane having selective permeability to ethanol in preference to the water. The vapors of ethanol having a lower boiling point are caused to permeate and diffuse through the membrane in preference to the water, to thereby increase ethanol concentration within the vacuum chamber **13**. Thus, ethanol of increased concentration can be recovered from the vacuum chamber **13**.

During the above separation process, the solution **S** is maintained at a raised temperature for enhancing evaporation of the component to be separated and therefore obtaining the intended component of increases concentration. To this end, a heating bath **16** is provided to surround the solution chamber **12**, as shown in FIG. 1. The heating of the solution may be effected alternatively by providing a heating jacket around the solution chamber **12**, providing an immersion heater in the solution chamber **12**, or the like heating device. The heating of the solution, in the present invention, means to heat the solution **S** to a temperature above that of an environment or room temperature and preferably that of the permeable membrane **14**. Thus, the heating is preferably carried out in such a manner as to raise the temperature of the solution **S** while maintaining the permeable membrane **14** at a lowered temperature. For positively providing a temperature difference between the solution **S** and the permeable membrane **14**, the apparatus of FIG. 1 includes a cooling jacket **18** surrounding the permeable membrane **14** for cooling the membrane **14** by a cooling medium continuously fed through the jacket **18**. By cooling the permeable membrane **14** while heating the solution **S**, a more enhanced separation could result. The exact reason or mechanism for such enhanced separation is not known, but it is assumed that the vapors of the components having a higher boiling point are most likely to aggregate when reaching adjacent the cooled membrane and return to liquid or at least form a large sized aggregation which is difficult to permeate through the membrane, thus enabling substantially only the vapors of the component having a lower boiling point to permeate through the membrane. It should be noted at this time that, although the apparatus of FIG. 1 is intended to cool the permeable membrane **14** and therefore the adjacent zones on the opposite sides thereof, the cooling could be limited only to the zone adjacent the solution side of the permeable membrane **14** for achieving the above enhanced separation, and the cooling is not necessarily made to the opposite zone in the vacuum chamber or the permeable membrane itself. Therefore, any other cooling scheme or means could be utilized to effect such limited cooling while retaining the equally enhanced separation.

The separation method of the present invention could be adapted for separation of a wide variety of solutions containing two or more liquid components, and is found particularly useful for separation of solutions which are difficult to be separated by conventional methods. Such useful adaptation includes, for example, separation of an alcohol, aromatic compounds or ether from an azeotropic mixture of water-alcohol, water-aromatic compound, or water-ether; separation of an organic solvent from a solution containing two or more organic solvents of differing but closing boiling points; separation of a structural isomer from a mixture containing two or more such isomers such as o-, m-, and p-xylenes; separation of an optical isomer from a mixture containing two or more such isomers such as dextro- and levo-rotary ones; separation of a mixture containing thermally decomposable or deformable substances such as medicines, living organisms, fruit juices, polymerizable monomers; recovery of a reaction product from a reaction system in equilibrium for accelerating the reaction; separation of volatile organic substances such as ammonia, amine, hydrogen sulfide, carbon dioxide, sulfur dioxide, or the mixture thereof from a waste liquid; separation of alcohols form a biomass; separation of an expensive organic solvent, e.g. dimethylfromamide, dimethylacetoamide, or dimethylsulfoxide from a spinning solution of synthetic fibers; separation of a solvent from a waste paint resulting in the preparation of a paint or in a paint processing line; separation of alcohols, organic solvents, or salts from an alcohol or solvent containing mixture resulting in a chemical plant; separation of trichlene from a dry-cleaning fluid; concentration of an emulsion solution; concentration and separation of ions of rare earth elements from a solution containing radio-active elements; concentration

4

of virus or bacteriophage; manufacture of sterile water or non-pyrogenic water for use in blood dialysis or other pharmaceutical or therapeutic purposes; and manufacture of ultrapure water for use in an electronic industry.

The present invention will be discussed with reference to the following examples, which are provided by way of illustration and not by way of limitation. All percent and parts are by weight.

Example 1

A 10 % aqueous solution of ethanol was separated by the use of a permeable membrane of polydimethylsiloxane which shows selective permeability to ethanol in preference to water. The polydimethylsiloxane membrane was prepared from the mixture of 7 parts of silicon (sold by SHINETSU KAGAKU, Japan as KE10), 0.25 parts of hardening agent (sold by the same as catalyst RA), and 49 parts of benzene. The mixture was stirred followed by placed onto a stainless-steel made membrane former and dried thereon at 25°C for 6 hours to obtain a 100 $\mu$ thick permeable membrane. Thus obtained membrane was fixed in the apparatus of FIG. 1 while the solution is placed in the solution chamber **12** out of direct contact with the membrane. The separating operations were carried out while keeping the vacuum chamber **13** at a reduced pressure of 1.5 x $10^{-2}$ Torr. and varying the temperatures of the heating bath **16** and the cooling jacket **18**, as listed in Table 1, to provide nine (9) samples of ethanol-rich solutions recovered through the membrane **14** with differing temperature conditions. The solution collected for 30 minutes after the start of separation was discarded and each sample was collected from the vacuum chamber **13** for 2 hours after an elapse of initial 30 minutes from the start of separation. Then, separation factor $\alpha$ and ethanol concentration for each sample (Nos. 1 to 9) were obtained as listed in Table 1. The separation factor $\alpha$ is defined by the following equation:

$$\alpha = \frac{Y_{ETOH}/Y_{H2O}}{X_{ETOH}/X_{H2O}}$$

wherein $X_{H2O}$ and $X_{ETOH}$ are fractions of water and ethanol respectively in aqueous ethanol solution placed in the solution chamber **12**, while $Y_{H2O}$ and $Y_{ETOH}$ are fractions of water and ethanol respectively for each sample solution collected in the vacuum chamber **13**. As apparent from the above relation, when $\alpha$ is greater than 1, it means that ethanol has passed through the membrane **14** in a greater amount than water and therefore that ethanol is allowed to pass preferentially through the membrane to a larger extent as the value $\alpha$ becomes greater.

Table 1

| samples | solution temp.(°C) | membrane temp.(°C) | concentration (wt%) | separation factor [$\alpha$] |
|---------|--------------------|--------------------|---------------------|------------------------------|
| No. 1 | 25 | 25 | 38.20 | 5.56 |
| No. 2 | 40 | 40 | 38.51 | 5.64 |
| No. 3 | 40 | 25 | 61.47 | 14.36 |
| No. 4 | 40 | 0 | 73.15 | 24.52 |
| No. 5 | 40 | -10 | 84.94 | 50.76 |
| No. 6 | 55 | 55 | 43.19 | 6.84 |
| No. 7 | 55 | 25 | 68.52 | 19.59 |
| No. 8 | 70 | 25 | 71.80 | 22.91 |
| No. 9 | 70 | 70 | 44.18 | 7.12 |

In Table 1, samples Nos. 1, 2, 6, and 9 were obtained by maintaining the solution and the permeable membrane at the same temperatures, while samples Nos. 3-5, 7, and 8 were obtained by maintaining the permeable membrane at a lower temperature than that of the solution. Each sample was analyzed to provide separation factor $\alpha$ and ethanol concentration, the results of which are shown in graphs of FIGS. 2, 3, and 4, in which a line marked by a triangle shows the concentration of each sample, a line marked by a rectangular shows the separation factor α of each sample, upper and lower figures in a brace indicate the solution temperature and membrane temperature, respectively.

FIG. 2 shows the graph for a first group of samples Nos. 1, 2, 6, and 9 in which the solution and the membrane were at the same temperatures. Sample No. 1 was obtained by leaving the solution and membrane at a room temperature of 25°C, and the other samples Nos. 2, 6, and 9 were obtained by raising the solution and membrane temperatures, respectively to 40, 55, 70°C. As seen from the figure that raising the solution temperature will results in a superior separation effect as confirmed both in ethanol concentration and separation factor α.

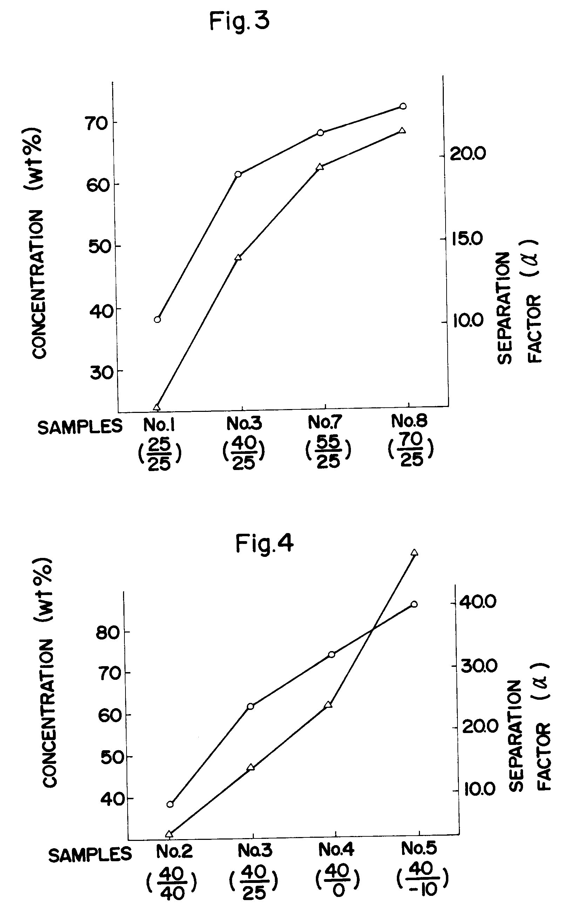

FIG. 3 shows the like graph for a second group of samples Nos. 1, 3, 7, and 8, which were obtained by fixing the membrane temperature at 25°C, while varying the solution temperature respectively to temperatures of 25, 40, 55, and 70°C. As seen from the figure, it is confirmed that samples Nos. 3, 7 and 8 obtained by raising the solution temperatures, respectively to 40, 55, 70°C exhibit superior separation results both in ethanol concentration and separation factor α, as compared to sample No. 1 in which no temperature difference was seen between the solution and membrane. Also noted from the figure is that the superior separation results are correspondingly enhanced as the solution temperature is raised.

FIG. 4 shows the like graph for a third group of samples Nos. 2, 3, 4, and 5, which were obtained by fixing the solution temperature at 40°C while varying the membrane temperature, respectively to 40, 25, 0, and -10°C. It is also confirmed from the figure that the separation effect is more enhanced as the temperature difference between the solution and the membrane is increased while fixing the solution temperature.

Example 2

A 10 % aqueous solution of methanol was separated in the same apparatus and by the same permeable membrane of polydimethylsiloxane as employed in Example 1. The like separating operations were carried out with a vacuum level of 2.0 x $10^{-2}$ Torr. in the vacuum chamber **13**, so as to obtain samples No. 10 and 11 with varying membrane temperatures but with a fixed solution temperature, as listed in Table 2. The samples were analyzed to provide the respective ethanol concentrations which are also listed in Table 2. From the above results, it is further confirmed that the separation effect also for ethanol is greatly enhanced by providing the temperature difference between the solution and the membrane.

Table 2

| samples | solution temp.(°C) | membrane temp.(°C) | concentration (wt%) |
|---------|---------------------|---------------------|----------------------|
| No. 10 | 40 | 40 | 35.50 |
| No. 11 | 40 | 5 | 72.50 |

LIST OF REFERENCE NUMERALS

10     vessel
12     solution chamber
13     vacuum chamber
14     permeable membrane
15     port
16     heating bath
18     cooling jacket
20     vessel
22     solution chamber
23     vacuum chamber
24     permeable membrane
30     vessel
32     solution chamber
33     vacuum chamber

EP 0 346 739 B1

34      permeable membrane

## Claims

1. A method for separating a liquid component from a solution containing two or more liquid components having different boiling points, comprising:

a) providing a vessel (10) divided by a permeable membrane (14) into a solution chamber (12) and a vacuum chamber (13), said membrane being selectively and preferentially permeable to the vapors of the component having a lower boiling point than those of the other components;

b) placing the solution (S) into the solution chamber out of direct contact with the permeable membrane; and

c) applying a vacuum to the vacuum chamber to evaporate the solution in the solution chamber and draw the resulting vapors through the permeable membrane into the vacuum chamber, whereby the components to be separated selectively permeates through the membrane in a vapor-to-vapor phase environment,

characterized in that

the solution in the solution chamber is heated to a temperature higher than the environment while said zone adjacent to the solution chamber side of said membrane or said membrane itself is cooled to a temperature lower than that of said solution in the solution chamber.

2. A method according to claim 1, characterized in that the solution is an aqueous solution of alcohol.

3. A method according to claim 2, wherein the permeable membrane is a polydimethylsiloxane membrane selectively and preferentially permeable to ethanol.

## Patentansprüche

1. Verfahren zum Abtrennen eines flüssigen Bestandteils von einer Lösung, die zwei oder mehr flüssige Bestandteile mit verschiedenen Siedepunkten enthält, welches umfaßt:

a) daß ein Behälter (10) vorgesehen ist, der durch eine durchlässige Membran (14) in eine Lösungskammer (12) und eine Vakuumkammer (13) unterteilt ist, wobei besagte Membran selektiv und bevorzugt durchlässig ist für die Dämpfe des Bestandteils mit einem niedrigeren Siedepunkt als für diejenigen der anderen Bestandteile;

b) daß die Lösung (S) in die Lösungskammer außer direktem Kontakt mit der durchlässigen Membran gegeben wird; und

c) daß an die Vakuumkammer ein Vakuum angelegt wird, um die Lösung in der Lösungskammer zu verdampfen und die resultierenden Dämpfe durch die durchlässige Membran in die Vakuumkammer zu ziehen, wodurch die zu trennenden Bestandteile selektiv durch die Membran in einer Dampf/Dampf-Phasenumgebung hindurchdringen,

dadurch gekennzeichnet, daß

die Lösung in der Lösungskammer auf eine höhere Temperatur als die Umgebung erwärmt wird, während besagte Zone benachbart zur Lösungskammerseite besagter Membran oder besagte Membran selbst auf eine niedrigere Temperatur als diejenige besagter Lösung in der Lösungskammer abgekühlt wird.

2. Verfahren nach Anspruch 1, dadurch gekennzeichnet, daß die Lösung eine wässrige Alkohollösung ist.

3. Verfahren nach Anspruch 2, wobei die durchlässige Membran eine Polydimethylsiloxan-Membran ist, die selektiv und bevorzugt durchlässig ist für Ethanol.

## Revendications

1. Procédé pour séparer un composant liquide d'une solution contenant deux ou plusieurs composants liquides ayant des points d'ébullition différents, consistant à

a) fournir un récipient (10) divisé par une membrane perméable (14) en une chambre à solution (12) et une chambre à vide (13), ladite membrane étant sélectivement et préférentiellement perméable aux vapeurs du composant ayant un point d'ébullition plus bas que ceux des autres composants ;

7

b) placer la solution (S) dans la chambre à solution hors d'un contact direct avec la membrane perméable ; et

c) appliquer un vide à la chambre à vide pour évaporer la solution contenue dans la chambre à solution et attirer les vapeurs résultantes dans la chambre à vide à travers la membrane perméable, si bien que les composants à séparer traversent sélectivement la membrane dans une ambiance de phase vapeur à phase vapeur,

caractérisé en ce que

la solution contenue dans la chambre à solution est chauffée à une température supérieure à celle de l'ambiance, tandis que la zone adjacente au côté tourné vers la chambre à solution de ladite membrane, ou ladite membrane elle-même, est refroidie à une température inférieure à celle de ladite solution contenue dans la chambre à solution.

2. Procédé selon la revendication 1, caractérisé par le fait que la solution est une solution aqueuse d'alcool.

3. Procédé selon la revendication 2, dans lequel la membrane perméable est une membrane de polydiméthylsiloxane sélectivement et préférentiellement perméable à l'éthanol.

Fig. I

Fig. 2

## Fig. 3

## Fig. 4

EP 0 346 739 B1

## Fig.5 (PRIOR ART)

## Fig.6 (PRIOR ART)